# EUROPEAN PATENT APPLICATION

(11) **EP 1 285 942 A1**
(43) Date of publication of application: **26.02.2003**
(21) Application number: 01932141.3
(22) Date of filing: 18.05.2001
(51) Int. Cl.: C08G 69/10, C07K 14/00, A61K 47/42, C12N 15/09, A61K 48/00

(54) **POLYPEPTIDE DERIVATIVES AND NUCLEIC ACID CARRIERS CONTAINING THE SAME**

(30) Priority: 18.05.2000 JP 2000146829
(71) Applicant: HISAMITSU PHARMACEUTICAL CO., INC., Tosu-shi Saga 841-0017 (JP)
(72) Inventor: GOTO, Takeshi Tsukuba Lab. Hisamitsu Pharm. Co Inc, Tsukuba-shi, Ibaraki 305-0856 (JP); NAKANISHI, Masaru Tsukuba Lab.Hisam. Pharm.Co Inc, Tsukuba-shi, Ibaraki 305-0856 (JP); YONEMURA, Keishi Tsukuba Lab.Hisam. Pharm.Co Inc, Tsukuba-shi, Ibaraki 305-0856 (JP); OOMORI, Naoya Tsukuba Lab. Hisamitsu Pharm. Co Inc, Tsukuba-shi, Ibaraki 305-0856 (JP); YASUKOUCHI, Takashi Tskuba Lab.Hisam. Pharm.Co Inc, Tsukuba-shi, Ibaraki 305-0856 (JP); OYA, Masanao, Tsukuba Lab. Hisamitsu Pharm. Co Inc, Tsukuba-shi, Ibaraki 305-0856 (JP)
(74) Representative: Grünecker, Kinkeldey, Stockmair & Schwanhäusser Anwaltssozietät
(86) International application number: JP0104176
(87) International publication number: WO01088017

(57) **Abstract**

There are disclosed polypeptide derivatives and their pharmaceutically acceptable salts represented by formula (1), as well as their utility as nucleic acid carriers in gene therapy.

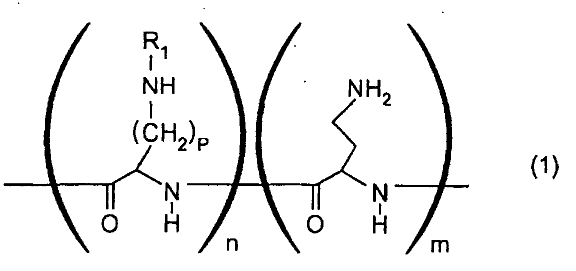

## Description

### Technical Field

This invention relates to nucleic acid carriers for transferring therapeutic genes into cells. More particularly, the invention relates to polypeptide derivatives obtained by modifying at least two side chain (free) amino groups of a polypeptide, which may contain polydiaminobutyric acid, with cell recognition groups as well as to their utility as nucleic acid carriers.

### Background Art

Retrovirus-mediated gene transfer is employed as the most effective means for gene therapy; however, with viral vectors there is a risk of infectious and immunologic reactions. Furthermore, the large-scale production of viral vectors is difficult, time consuming and requires special facilities. For these reasons, synthetic carriers which will replace the viral vectors have been studied in the past. One example is that JPA 55-500053 (1980), EP0775751 and EP0834572 disclose attempts to use polydiaminobutyric acid as a carrier for therapeutic genes. In the disclosure of the publication of JPA 55-500053, a covalent bond is utilized as a means with which polydiaminobutyric acid retains the therapeutic gene. However, in this case it is necessary to liberate (or separate) the therapeutic gene from polydiaminobutyric acid, i.e., the carrier, when the gene has been incorporated into the cell, which is not desired. In EP0775751 and EP0834572, oligodiaminobutyric acid is employed, where a physiologically active substance (cell recognition factor etc.) that modifies the oligodiaminobutyric acid is conjugated thereto only at its terminus. Since the physiologically active substance only provides a low level of effect, an adjuvant such as a phospholipid, which is referred to as "helper lipid" will be needed in addition. When the terminus of a branched oligodiaminobutyric acid is modified with the physiologically active substance, a positive charge does not appear on the surface because the electric bonding of an anionic therapeutic gene is prevented. This will result in low incorporation into the cell.

In addition to the synthetic carriers illustrated above, polylysine has also been studied for its use as a carrier (J. Controlled Release, vol. 53, 1998, p301-310). However, complexes comprising polylysine as a carrier tend to form precipitates with their increasing concentrations, and thus they find difficulties in being used in the actual gene therapies. Especially when a drug solution containing particles that likely form precipitates in veins is administered, it can be the cause responsible for the blockade of blood vessels, thrombus and the like. Even in the case of local administration, there is a problem such as the clogging of syringe needles; and another problem is that it is not possible to transfer into the target cell, a gene in an amount sufficient to carry out treatment.

Under these circumstances, there is a strong need for the research and development on a non-viral carrier of a therapeutic gene that can be introduced into the living body safely and efficiently and that can cause the action of the therapeutic gene to be adequately manifested (which will be referred to as "nucleic acid carrier(s)" hereafter), as well as on a gene therapeutic composition utilizing the same.

### Disclosure of the Invention

It is an object of this invention to provide a nucleic acid carrier for the efficient and safe transfer of a nucleic acid or the like (a therapeutic gene) into a cell. Particularly, it is an object of the invention to provide a synthetic carrier that accomplishes the efficiency of gene transfer which is not inferior to that with viral vectors and that does not accompany side reactions such as the blockade of blood vessels.

As a result of having pursued diligent studies to solve the above-stated problems, it was discovered that when a polypeptide derivative of which at least two side chain (free) amino groups are modified with cell recognition groups and which may contain polydiaminobutyric acid was used as a nucleic acid carrier for a therapeutic gene, the therapeutic gene could be transferred into a cell efficiently and safely. Thus, this has led to the completion of the invention.

In addition, during the production of the polypeptide derivative, it was discovered that when the reaction system at the stage where the 4-amino group of diaminobutyric acid would be selectively protected with a carbobenzoxy group was maintained within a pH range of from 8 to 11 and the amino group was allowed to react with [p-(benzyloxycarbonyl)phenyl]dimethylsulfonium methylsulfate (Z-DSP), a protected form of diaminobutyric acid could be obtained in a high yield and with high purity. Thus, this has led to the completion of the invention.

Specifically, this invention provides a peptide derivative represented by formula (I): 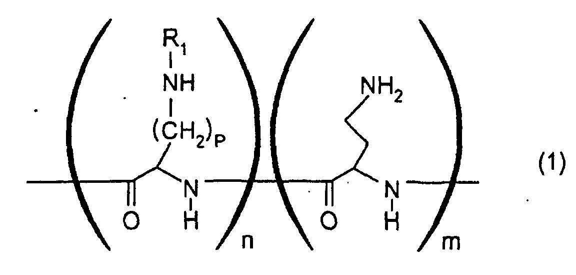 wherein R₁ is derived from one member selected from the' group consisting of a monosaccharide, an oligosaccharide, a polysaccharide, a peptide, an oligopeptide, a polypeptide, an antibody and a cell-specific ligand, or is selected from the group consisting of an optionally substituted C₁-C₂₀ alkyl, an optionally substituted C₁-C₂₀ alkenyl, an optionally substituted C₇-C₄₅ aryl, an optionally substituted C₇-C₄₅ alkylaryl, an optionally substituted C₇-C₄₅ alkenylaryl, and an optionally substituted amino group wherein the substituent may be a moiety of the one member selected from the group consisting of a monosaccharide, an oligosaccharide, a polysaccharide, a peptide, an oligopeptide, a polypeptide, an antibody and a cell-specific ligand; "p" represents an integer of 1-5; "m" represents an integer of 2 or more; and "n" represents an integer of 2 or more, provided that "m + n" represents an integer of 300 or less,
or a pharmaceutically acceptable salt thereof.

In the polypeptide derivative or a pharmaceutically acceptable thereof as described above, R₁ is preferably a radical represented by the formula of R₂-L-, wherein "L" is a linker selected from carbonyl, thiocarbonyl, imine or methylene which may further contain one or more methylene groups; and R₂ is a residual portion of the R₁ radical defined previously.

More preferably, L is a linker containing imine which may further contain one or more methylene groups; thus R₁ is a radical represented by formula (2) wherein "X" is 0 or a natural number of 20 or less; and then the polypeptide derivative is represented by formula (3). A pharmaceutically acceptable salt of the polypeptide derivative represented by the formula (3) is also particularly preferred.

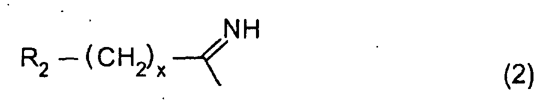

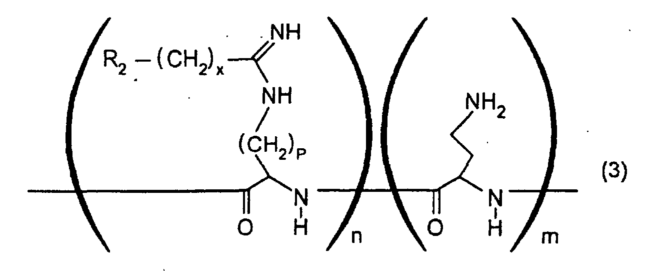

Most preferably, "p" is 2 in the formula (3) and thus the derivative is represented by formula (4). A pharmaceutically acceptable salt of the polypeptide derivative represented by the formula (4) is also most preferred.

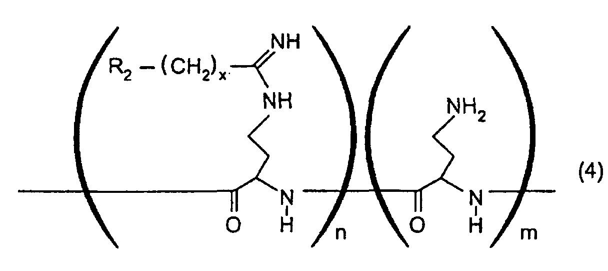

In the polypeptide derivative or a pharmaceutically acceptable salt thereof as described above, it is particularly preferred that R₂ be a moiety derived from one member selected from the group consisting of a monosaccharide, an oligosaccharide, a polysaccharide, a peptide, an oligopeptide, a polypeptide, an antibody and a cell-specific ligand.

In the polypeptide derivative or a pharmaceutically acceptable salt thereof as described above, it is more preferred that R₂ be a thioglycosyl group, an O-glycosyl group or a N-glycosyl group each of which is derived from one member selected from the group consisting of a monosaccharide, an oligosaccharide and a polysaccharide.

According to this invention, particularly preferred individual polypeptides are represented by formula (5). Pharmaceutically acceptable salts of the polypeptide derivatives represented by the formula (5) are also particularly preferred.

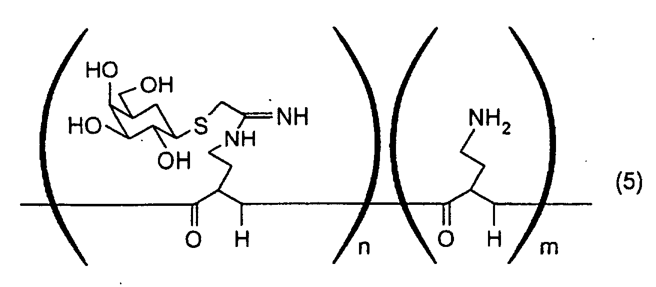

This invention also provides a nucleic acid carrier comprising the polypeptide derivative represented by the formula (1) or a pharmaceutically acceptable salt thereof.

This invention further provides a gene therapeutic composition comprising the nucleic acid carrier and a therapeutic gene.

In one aspect of the invention, there is provided a method of selectively protecting the 4-amino group of diaminobutyric acid with a carbobenzoxy group, comprising reacting diaminobutyric acid or a acid addition salt thereof with [p-(benzyloxycarbonyl)phenyl]dimethylsulfonium methylsulfate in a reaction inert medium at a pH of from 8 to 11.

### Brief Description of the Drawings

Fig. 1 is a reaction scheme showing an illustration of the synthetic pathway for the diaminobutyric acid modified with galactose at its side chain which is an example of the polypeptides according to this invention.
Fig. 2 is a graph showing the results of measurement of the activity of luciferase expressed arising from the gene transfer into HepG cells according to this invention.
Fig. 3 is a graph showing the results of measurement of the activity of luciferase expressed arising from the gene transfer into mice according to this invention.

### Best Mode for Carrying Out the Invention

This invention relates to a polypeptide derivative which is useful as a nucleic acid carrier for carrying a therapeutic gene and which may contain polydiaminobutyric acid, wherein at least two side chain (free) amino groups are modified with cell recognition groups.

The polypeptide of this invention is a polypeptide represented by the formula (1) or a pharmaceutically acceptable salt thereof. Where there is no need to describe the polypeptide derivative and its pharmaceutically acceptable salt discriminatingly, either or both of them are expressed by the term, "polypeptide(s) of this invention."

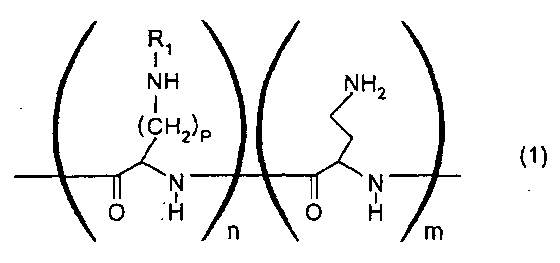

In the formula (1) R₁ is derived from one member selected from the group consisting of a monosaccharide, an oligosaccharide, a polysaccharide, a peptide, an oligopeptide, a polypeptide, an antibody and a cell-specific ligand, or is selected from the group consisting of an optionally substituted C₁-C₂₀ alkyl, an optionally substituted C₁-C₂₀ alkenyl, an optionally substituted C₇-C₄₅ aryl, an optionally substituted C₇-C₄₅ alkylaryl, an optionally substituted C₇-C₄₅ alkenylaryl, and an optionally substituted amino group wherein the substituent may be a moiety of the one member selected from the group consisting of a monosaccharide, an oligosaccharide, a polysaccharide, a peptide, an oligopeptide, a polypeptide, an antibody and a cell-specific ligand; "p" represents an integer of 1-5; "m" represents an integer of 2 or more; and "n" represents an integer of 2 or more, provided that "m + n" represents an integer of 300 or less.

As used herein, "alkyl" means an aliphatic group (straight chain, branched chain, cyclic chain or a combination of the foregoing) having the above-defined carbon number.

As used herein, "alkenyl" means an unsaturated aliphatic group (straight chain, branched chain, cyclic chain or a combination of the foregoing) having the above-defined carbon number.

As used herein, "aryl" means an aromatic ring having the above-defined carbon number which may be substituted with one to four substituents. Here, the substituents include, but not limited to C₁-C₆ alkyl, C₁-C₆ alkoxy, C₁-C₆ alkylamino, hydroxyl, cyano, halogen, mercapto, nitro, carboxyl, formyl, thioalkoxy, and the like.

As used herein, "alkylaryl" and "alkenylaryl" mean the alkyl group and the alkenyl group as defined above each of which is bonded to one to three aryl groups. Similarly to the above-mentioned aryl group, this aryl portion may be substituted with one to four substituents.

Any of the "alkyl group," "alkenyl group," "aryl group," "alkylaryl group," and "alkenylaryl group" as defined above may optionally contain as part of its structure, a substituent corresponding to the linker (L) which will be described later.

As used herein, "pharmaceutically acceptable salt" means a salt (or salts) formed from the organic or inorganic acid as illustrated below with the free amino group(s) (which, in other words, is not utilized for the backbone peptide bonding) or the free imino group(s) of the polypeptide derivative of the formula (1) that retains the biological utility of the polypeptide derivative itself (i.e., as a nucleic acid carrier) and that is non-toxic biologically or physiologically and does not exhibit undesired side reactions upon administration to the human body. Therefore, it is not particularly limited insofar as it is pharmaceutically acceptable. Preferably mentioned are the salt from an inorganic acid such as boric acid, hydrochloric acid, sulfuric acid, nitric acid, or phosphoric acid and the salt from an organic acid such as acetic acid, propionic acid, citric acid, lactic acid, oxalic acid, succinic acid, tartaric acid, malonic acid, fumaric acid, or malic acid. Such pharmaceutically acceptable salts may be readily prepared from the polypeptide derivatives of this invention according to standard methods.

A polypeptide peptide of the formula (1) comprises a structural unit A that may contain polydiaminobutyric acid of which one amino group is blocked with R₁ radical and a structural unit B comprising polydiaminobutyric acid having an free amino group (formula (6)). When the polypeptide derivative is a polymer, the formula (1) represents a block copolymer consisting of the structural unit A and the structural unit B, or alternatively a random copolymer consisting of the structural unit A and the structural unit B. In this invention, the random copolymer is particularly preferred. The polypeptide derivatives represented by the formulae (3) to (5) are random polymers. Furthermore, the polypeptide derivative comprises the structural units A and B in any proportions; and they may exist in the form of their respective pharmaceutically acceptable salts described above. The polypeptide derivative may either be that which has a single 'molecular weight or an aggregate having molecular weight distribution.

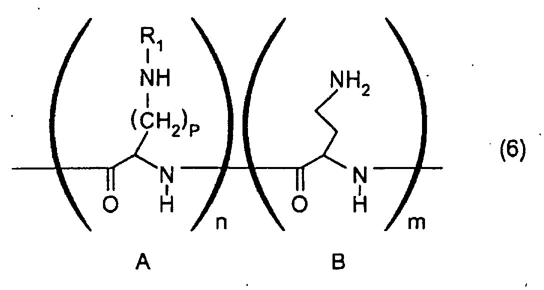

In the structural units A and B, each unit can exist as D-form or L-form which is an optical isomer. Thus, the polypeptide derivative of this invention is a polypeptide of D-form or of L-form, or a polypeptide that is their mixture in any proportion.

In the formula (1), "p" is an integer of from 1 to 20, and preferably it is 2. In this case the structural unit A comprises the polymer of diaminobutyric acid (which will be referred to as "DBA" hereafter) as a base unit. The polypeptide derivative of the formula (1) will contain polydiaminobutyric acid (which will be referred to as "pDBA" hereafter).

By "the number of residue of a polypeptide derivative according to this invention" is meant "m+n" in the formula (1). Here, "m" represents an integer of 2 or more and "n" represents an integer of 2 or more. The preferred number of residues is at least 20 residues. More preferably, the number of residues is at least 25, and most preferably at least 30. The preferred number (m+n) of residues of the polypeptide derivative according to the invention is 300 or less. Preferably, the number of residues is 280 or less; and more preferably the number is 250 or less. When the number of residues is too large, the synthesis of polypeptide derivatives will be difficult and handling will also be inconvenient. On the other hand, when the number of residues is too small, performance when used as a nucleic acid carrier will be inadequate. Further, the preferred value for m is 3 or more, and more preferred value is 4 or more.

R₁ as defined above is a radical to block the free amino group and is derived from one member selected from the group consisting of a monosaccharide, an oligosaccharide, a polysaccharide, a peptide, an oligopeptide, a polypeptide, an antibody and a cell-specific ligand, or is selected from the group consisting of an optionally substituted C₁-C₂₀ alkyl, an optionally substituted C₁-C₂₀ alkenyl, an optionally substituted C₇-C₄₅ aryl, an optionally substituted C₇-C₄₅ alkylaryl (aralkyl), an optionally substituted C₇-C₄₅ alkenylaryl, and an optionally substituted amino group.

The R₁ radical according to this invention has two characteristics principally. One of them is cell recognition function and this allows the R₁ radical to recognize a component of the targeted cell (especially, an antigen or a receptor on the cell surface). Specifically, the introduction of R₁ radical imparts a cell recognition site to the polypeptide derivative of this invention. Therefore, when the polypeptide derivative of this invention is used as the nucleic acid carrier, it will be able to efficiently deliver a therapeutic gene to the targeted cell. A wide variety of naturally occurring factors or synthetic compounds are known as the molecules possessing the cell recognition function. A Moiety derivable from those known molecules may preferably be utilized as the R₁ radical in this invention. Preferably, they are monosaccharides, oligosaccharides, polysaccharides, peptides, oligopeptides, polypeptides, antibodies and cell-specific ligands, etc. The illustrated concrete substances include, but not limited to galactose, mannose, glucose, fructose, lactose, dextran, Gal-GlccNAc, RGD, transferrin, LDL (low density lipoprotein), asialoorosomucoid and the like.

The other characteristic of the R₁ radical is that its introduction will render the polypeptide derivative more cationic. This is necessary to bring electrostatic interaction with the therapeutic gene such as a nucleic acid that is normally charged negatively and to incorporate the gene into a composition. For this purpose, the R₁ radical may be provided with suitable substituents, if desired. The representative substituents include, *inter alia,* alkoxy, alkylthio, alkylamino, amino, imino, guanigino, and hydrazino groups.

In a preferred embodiment of this invention, R₁ is represented by the formula R₂-L-. Here, L is a linker bonded to the free amino group. Suitable linkers include, but not limited to carbonyl, thiocarbonyl, imine and methylene. The linker may contain one or more methylene groups in addition to those mentioned above.

The particularly preferred example of the linker contains imine. In this case R₁ is represented by the formula (2). 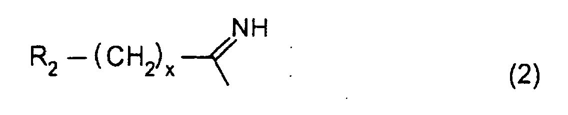 wherein "X" is 0 or a natural number of 20 or less.

Furthermore, the polypeptide derivative of this invention is represented by the formula (3).

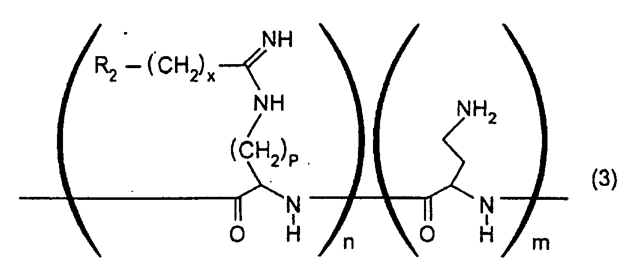

Within the preferred group of the polypeptide derivatives of this invention represented by the formula (3) in which "L" contains imine, the particularly preferred group of the polypeptide derivatives are compounds wherein "p" is 2, which are represented by the formula (4).

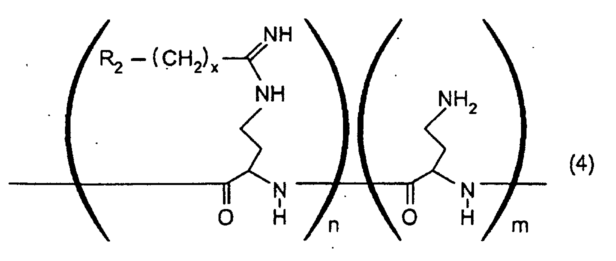

Another preferred group of the polypeptide derivatives are compounds wherein R₂ is a thioglycosyl group, an O-glycosyl group or a N-glycosyl group each of which is a moiety derived from one member selected from a monosaccharide, an oligosaccharide, or a polysaccharide.

According to an embodiment of the invention, the particularly preferred individual polypeptide derivative is represented by the formula (5).

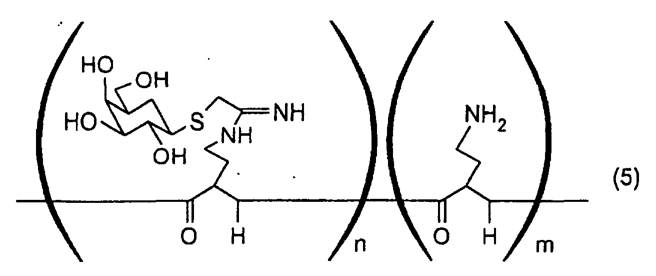

### (Synthetic Method for Polypeptide Derivatives)

The polypeptide derivatives of the formula (1) can be synthesized by combining several reactions that are known to one skilled in the art. The method employed in this invention comprises the syntheses of the structural units A and B represented by the formula (6) and the formation of peptide bonds from the respective structural units synthesized; and it specifically includes the steps described below. 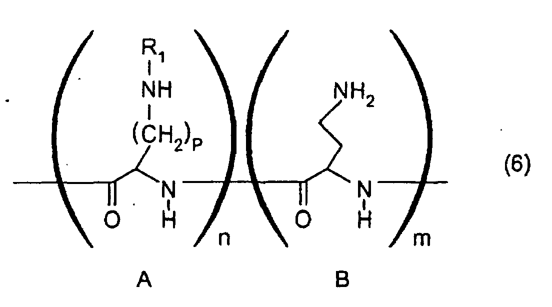
Step 1: the synthesis of Fmoc amino acid (oligo/polypeptide) for solid phase synthesis which corresponds to the structural unit A
Step 2: the synthesis of Fmoc amino acid (oligo/polypeptide) for solid phase synthesis which corresponds to the structural unit B
Step 3: solid phase peptide synthesis using the Fmoc amino acids (oligo/polypeptide) synthesized in steps 1 and 2 (ligation)

### Each step will be described in detail below.

### [Step 1]

(1-1) A solution of a monomer (polymer) having an amino group at its side chain is treated with a base such as the hydroxide or the carbonate of an alkaline metal and the pH of solution is adjusted. A protecting reagent R-X (R is Z, Boc, Troc, Z(Cl) or the like and X is a leaving group) activated with the suitable leaving group (such as halogen, sulfonate, phenoxide or derivatives of the foregoing) is allowed to react with the solution, introducing the protecting group to the side chain amino group. In this case the starting polymer for the preparation of the polymer preferably employs diaminobutyric acid, lysine, ornithine, or diaminopropionic acid. The monomer (polymer) into which the protecting group has been introduced is normally obtained as a precipitate. Water and a volatile organic solvent that is miscible with water in any proportion, or alternatively diethyl ether or ethyl acetate may be used to wash these precipitates.

(1-2) The compound prepared in 1-1 is dissolved in water or an organic solvent that is miscible with water in any proportion. The solution is treated with a base such as the hydroxide or the carbonate of an alkaline metal and the pH of solution is adjusted. To this is added an Fmoc reagent such as 9-fluorenylmethylchloroformate or N-(9-fluorenylmethoxycarbonyloxy)succinimide to allow for reaction. The resulting precipitates are washed with water and a volatile organic solvent that is miscible with water in any proportion, or alternatively with diethyl ether or ethyl acetate and dried.

(1-3) The side chain amino group of the compound obtained in 1-2 is removed by an appropriate method. Then, the amino group is allowed to react with a glycosyl halide, an alkyl halide having a protected peptide at its side chain, or the like. An Fmoc amino acid (oligo/polypeptide) derivative is thus synthesized where the side chain amino group is blocked with the R₁ radical described above.

### [Step 2]

(2-1) A solution of diaminobutyric acid having a free amino group at its side chain is treated with a base such as the hydroxide or the carbonate of an alkaline metal and the pH of solution is adjusted. A protecting reagent R-X (R is Z, Boc, Troc, Z(Cl) or the like and X is a leaving group) activated with the suitable leaving group (such as halogen, sulfonate, phenoxide or derivatives of the foregoing) is allowed to react with the solution, introducing the protecting group to the side chain amino group. DBA into which the protecting group has been introduced is normally obtained as a precipitate. Water and a volatile organic solvent that is miscible with water in any proportion, or alternatively diethyl ether or ethyl acetate may be used to wash these precipitates.

(2-2) The protected DBA as prepared in 2-1 is dissolved in water and an organic solvent that is miscible with water in any proportion. The solution is treated with a base such as the hydroxide or the carbonate of an alkaline metal and the pH of solution is adjusted. To this is added an Fmoc reagent such as 9-fluorenylmethylchloroformate or N-(9-fluorenylmethoxycarbonyloxy)succinimide to allow for reaction. The resulting precipitates are washed with water and a volatile organic solvent that is miscible with water in any proportion, or alternatively with diethyl ether or ethyl acetate and dried.

### [Step 3]

(3-1) Fmoc amino acids (oligo/polypeptides) for peptide synthesis as prepared in Steps 1 and 2, Fmoc DBA and Wang resin are used to synthesize a protected polypeptide in solid phase according to Fmoc method. The protected polypeptide is subjected to deresinification and deprotection following appropriate methods, producing the polypeptide derivative of this invention. For detailed experimental conditions, see Jikken Kagaku Koza (Experimental Chemistry Monograph Series), 4th Edition, Vol. 22, p 295-306.

To fully describe the modifications of the synthetic method described above that are practiced in this invention (which will be referred to as "the synthetic method according to this invention" hereafter), a specific example of the synthesis of the polypeptide derivative represented by the formula (4) will be illustrated.

To synthesize the polypeptide derivative of the formula (4) (polydiaminobutyric acid derivative), preferably the side chain amino group (4-position) of diaminobutyric acid is protected with a suitable protecting group and the amino acid moiety is converted into an acid anhydride thereof using triphosgene, after which it is used as a monomer for polycondensation. The use of this monomer will enable a suitable initiator to be used in the polycondensation reaction and a preferable number of monomers to be introduced.

The method for synthesizing polydiaminobutyric acid involves three steps as described below.

The first step is one in which the amino group at the 4-position of DBA is protected; the second step is one in which diaminobutyric acid anhydride (which will be abbreviated as "NCA") is produced from the selectively protected DBA(R); and the third step is one in which pDBA is synthesized from NCA. Here, the "R" in DBA(R) means the protecting group introduced into the 4-amino group.

The amino group in the side chain (4-position) of diaminobutyric acid first needs to be selectively protected. Conventionally, the copper complex method (Jikken Kagaku Koza-Experimental Chemistry Monograph Series Vol. 22, Ed. The Chemical Society of Japan, p239) and the direct protection method (Jikken Kagaku Koza-Experimental Chemistry Monograph Series Vol. 22, Ed. The Chemical Society of Japan, p238) are known as the methods of choice. Since the copper complex method employs a heavy metal, copper, and requires a number of steps, it is not an industrially advantageous reaction pathway; and the direct protection method is more convenient. However, there has been no report that the latter method is utilized to selectively protect the side chain amino acid of diaminobutyric acid.

According to the first step of the synthetic method of this invention, a DBA solution is treated with a base such as the hydroxide or the carbonate of an alkaline metal and the pH of solution is adjusted. A protecting reagent R-X (R is Z, Boc, Troc, Z(Cl) or the like and X is a leaving group) activated with a suitable leaving group (such as halogen, sulfonate, phenoxide or derivatives of the foregoing) is allowed to react with the solution, introducing the protecting group to DBA. The pH of the reaction system is preferably from 8 to 11, more preferably from 8.5 to 10.5, and most preferably 9.2 to 9.8. DBA(R) into which the protecting group has been introduced is subjected to after-treatment following a standard method. By using the direct protection method, the protection reaction is carried out within a predetermined pH range to selectively protect the amino acid of DBA, which has not been hitherto known to one skilled in the art. Accordingly, the selective protection method of amino groups constitutes one aspect of this invention.

In the second step, DBA(R) is suspended in a suitable solvent and is allowed to react with phosgene, phosgene dimmer, or triphosgene, yielding NCA. The solvent to be used is appropriately a solvent for which NCA (the product) has high solubility and which does not react with NCA. Specifically, there are mentioned THF, acetonitrile, ethyl acetate, dichloromethane, etc. For the crystallization of the produced NCA, the suitable solvent is that which does not react with NCA and which has little hygroscopic property, including diethyl ether, diisopropyl ether, hexane, toluene, etc.

In the third step, NCA is dissolved in a suitable solvent and an appropriate initiator is allowed to act thereon, yielding a polymer. The solvent to be used is appropriately a solvent the dissolution power of which is high for NCA but low for the product (or a polymer). Specifically, there are mentioned THF, acetonitrile, ethyl acetate, dichloromethane, etc. Usable as the initiator are amines, alcohols, alkoxides, etc; and 'alkylamines, particularly butylamine, are preferred. The polymer is produced as a precipitate. An inert solvent that does not dissolve polymer is used, which includes acetonitrile, diethyl ether, hexane, ethanol, methanol, etc. Depending on the nature of the protecting group, a suitable method may be used to remove the protecting group from the produced polymer. Standard methods such as dialysis and ultrafiltration may be used for the desalting (after deprotection) and the elimination of low molecular weight impurities.

The synthesis of the polypeptide derivatives of the formula (4) can be accomplished by modifying two or more free amino groups at the 4-position of polydiaminobutyric acid as obtained above with a modifying group (s) (which corresponds to R₂-(CH₂)ₓ-(C=NH)- in the formula). The modifying group to be introduced into pDBA is first derived from its parent substance (compound) and is activated so as to be reactive to the amino group(s). Although the method of activation may differ with respect to the nature of the modifying group, it can appropriately be chosen from techniques known to one skilled in the art. Subsequently, the activated form of the modifying group and the 4-amino group of polydiaminobutyric acid are subjected to coupling reaction, yielding the objective modified peptide derivative.

According to a preferred embodiment of this invention, a metal alkoxide is allowed to act on the parent substance (such as a saccharide or a protein) having a nitrile group already introduced, in a suitable solvent. The nitrile group is thereby converted into an alkoxyimmino group, where the aforementioned activation is carried out. Here, the metal to be used in the alkoxide is preferably an alkaline metal and sodium is particularly preferable. The alcoholic solvent is preferably a lower alcohol and particularly, methanol or ethanol is preferable.

The activated alkoxyimino group is then allowed to react with the 4-amino group of polydiaminobutyric acid under basic conditions (pH 8 to 11), when there is produced the polypeptide derivative of the formula (4) the 4-amino group of which has been modified with the modifying group that will be a tissue recognition site. The buffer to be used in the coupling reaction employs that which has buffering capability at the aforementioned pH and which does not contain any amine component. Especially, borate buffer is preferable. After the coupling reaction is complete, the desalting of the polypeptide derivative (the final product) and the elimination of low molecular weight impurities can be carried out by dialysis or ultrafiltration as previously described.

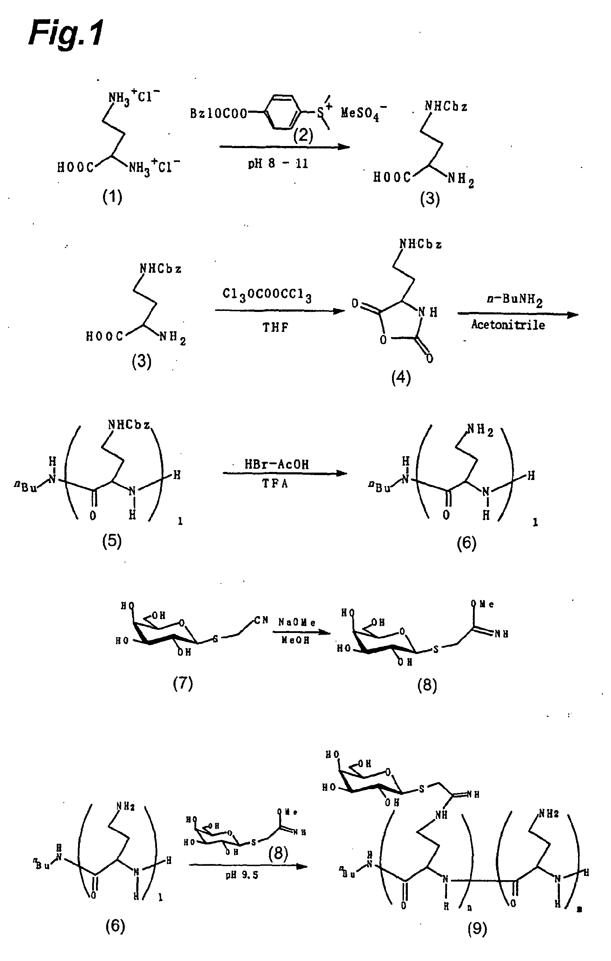

The thus obtained polypeptide derivatives of this invention are useful as a nucleic acid carrier to deliver therapeutic genes. By way of an example using the polypeptide derivative of this invention modified with galactose (compound of the formula (5)), the whole synthetic process for the polypeptide derivatives of this invention (as has been described above) is shown in Fig. 1. The detail of each step will be described in Examples.

### (Detection of Nucleic Acid Carrier)

The structure of the nucleic acid carrier comprising the polypeptide derivative of this invention has such structural characteristics as have been previously described for the polypeptide derivative. Therefore, it will be possible to detect the nucleic acid carrier of this invention based on such structural characteristics. Even if the nucleic acid carrier of this invention itself, or a gene therapeutic composition comprising it are used in different forms, the detection of nucleic acid carrier of this invention will be likewise possible when suitable pretreatment is performed. One skilled in the art can readily select the necessary treatment for this purpose.

The method of detection is not particularly limited; and a variety of ordinarily known polypeptide analysis methods can be used (J. Controlled Release vol. 54, 1998, p39-48). Specifically usable are the qualitative and quantitative analysis of diaminobutyric acid following the amino acid assay method in peptides, the determination of the number of residues based on molecular weight measurement using various kinds of liquid chromatography, mass spectroscopy, and chemical qualitative analysis methods.

### (Gene Therapeutic Composition and Therapeutic Gene)

The gene therapeutic composition of this invention comprises at least a nucleic carrier of the invention and a therapeutic gene. As used herein, "therapeutic gene" means various nucleic acids or nucleotide derivatives as will be described below.

The nucleic acid and the therapeutic gene can form a complex at different ratios to prepare the gene therapeutic composition. If the ratio of therapeutic gene to nucleic acid carrier (weight (w)/weight (w)) is in the range of from 2:1 to 1:50, therapeutic effects can be obtained. More preferably, if compounding is carried out at the ratio of therapeutic gene to nucleic acid carrier (w/w) being in the range of from 1:1 to 1:30, there can be obtained even more effectiveness. If the ratio of therapeutic gene to nucleic acid carrier (w/w) is 1:50 or more, the quantity of the free nucleic acid that does not participate in the complex with the therapeutic gene will increase, which is undesirable. In contrast, if the ratio is 2:1 or less, it will be undesirable because the affinity to the cell surface of the cell into which the gene is transferred decreases, causing the efficiency of therapeutic gene transfer to be lowered.

As stated previously, the nucleic acid carrier of this invention can have a positive charge and thus can retain the therapeutic gene, such as nucleic acid, principally having a negative charge through electrostatic bonding. Consequently, after the carrier is delivered to or within the objective cell, it will be possible to efficiently liberate the therapeutic gene and to cause it to be expressed. It will also be possible to control this action by adequately selecting the charge ratio of therapeutic gene to nucleic acid carrier. In this invention, if the charge ratio is set in the range of from 1:1 to 1:40, greater effects will be obtained. If the charge ratio is 1:1 or less, the affinity to the cell surface will decrease, causing the efficiency of therapeutic gene transfer to be lowered. In contrast, if it is 1:40 or more, there will be an increased quantity of the free nucleic acid carrier that does not participate in the complex with the therapeutic gene, which is undesirable.

As to the therapeutic genes to be transferred into the cell by the nucleic acid carrier of this invention, their nucleic acids or nucleotide derivatives are not particularly limited with respect to the kinds, molecular weights, shapes, and the sequences of genes encoded. Specifically, the molecular weight of nucleic acid is non-limiting such that it may be from approximately 20 bases of oligonucleotide to several kilo-bases of cosmid gene. For the shape of nucleic acid, a single-stranded gene, a double-stranded gene, a triple-stranded forming gene, DNA, RNA, a DNA/RNA chimera-type gene, a phosphothioate-type gene, a straight-chain gene, a circular gene, and the like may be used without any restriction. The sequence of gene to be encoded can employ, in addition to the therapeutic gene, any sequence among a promoter or an enhancer for the transcription of the therapeutic gene, a poly-A signal, a marker gene for labeling and/or selecting the cell into which the gene has been transferred, a virus-derived gene sequence for the efficient insertion of gene into cellular genomic DNA sequences, and a signal sequence for extracellularly secreting the substance that acts as drug and/or for having the substance remained at localized sites within a cell.

For the therapeutic genes, genes corresponding to disorders, namely genes that act against the disorders in an antagonistic manner or genes that supplement those lacking in the disorders may be used. Specifically, there are mentioned SOD, antiinflammatory cytokines, and genes encoding the peptides that act on cell-adhesion factors in an antagonistic manner for inflammatory disorders; genes encoding normal enzymes for enzyme-deficient disorders; genes encoding normal receptors for receptor-deficient disorders; thymidine kinases that kill virus-infected cells, genes encoding toxins such as diphtheria toxin, genes encoding antisense, triplehelixes, ribozymes, decoys, and transdominant mutants all of which inhibit the replication of viruses for virus infections; thymidine kinases that kill cancer cells, genes encoding toxins such as diphtheria toxin, genes encoding antisense, triplehelixes, and ribozymes all of which inactivate cancer genes, cancer-suppressing genes such as p53 that normalize the cancer cells, genes encoding antisense, triplehelixes, and ribozymes all of which inactivate genes that are involved in the multidrug resistance against anti-cancer agents for cancers; and genes encoding LDL receptors for familial hypercholesterolemia.

As for the expression cassettes to be used for the therapeutic gene, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, and most preferably expression cassettes capable of gene expression in the cells derived from a human. The gene promoters that can be used as expression cassettes include: for example, virus-derived promoters from an Adenovirus, a cytomegalovirus, a human immunodeficiency virus, a simian virus 40, a Rous sarcoma virus, a herpes simplex virus, a murine leukemia virus, a sinbis virus, a Sendai virus, a hepatitis type A virus, a hepatitis type B virus, a hepatitis type C virus, a papilloma virus, a human T cell leukemia virus, an influenza virus, a Japanese encephalitis virus, a JC virus, parbovirus B19, a poliovirus, and the like; mammal-derived promoters such as albumin and a heat shock protein; and chimera type promoters such as a CAG promoter.

As for a signal sequence for extracellularly secreting the drug encoded by the therapeutic gene and/or for having the substance remained at localized sites within a cell, there may used a signal peptide derived from interleukin-2 that assists extracellular secretion (Fusao Komada, The 115th Annual Meeting of The Pharmaceutical Society of Japan, Abstracts, 4, 12, 1995), a peptide derived from Adenovirus E1a that promotes nuclear localization, a peptide derived from polyoma virus large T antigen, a peptide derived from SV40 large T antigen, a peptide derived from nucleoplasmin, and a peptide derived from the HTLV1p24 post-transcription regulating protein (Kalderon, D., et al., Cell, 39, 499, 1984).

The gene therapeutic composition of this invention may be prepared by mixing the therapeutic gene that has been designed for therapeutic purposes as illustrated above and the nucleic acid carrier. More specifically, after the nucleic acid carrier and the therapeutic gene that has been designed for therapeutic purposes are separately dissolved in an appropriate solvent such as water, physiological saline water, or an isotonicated buffer, they are admixed and allowed to stand for 10 minutes to 30 minutes, thus enabling the preparation. Here, the ratio of the nucleic acid constituting the therapeutic gene and the nucleic acid carrier is not particularly limited; but the nucleic acid carrier is used at a level of from about 0.5 µg to 50 µg, and preferably from 1 µg to 30 µg relative to 1 µg of the nucleic acid.

### (Methods of Use of the Gene Therapeutic Composition)

The gene therapeutic composition of this invention can be used in the gene therapy through autologous implantation (*ex vivo* gene therapy) where the target cells are first removed outside the body from the patient and the cells are then returned to the body of the patient after the therapeutic gene has been transferred into the cells. The therapeutic gene can also be used in the gene therapy where the therapeutic gene is directly administered to the patient (*in vivo* gene therapy).

Gene therapy is largely classified into "Augmentation Gene Therapy" in which aberrant (causative) genes are left intact and new (normal) genes are augmented and "Replacement Therapy" in which aberrant genes are replaced with normal genes. The nucleic acid carriers of this invention can be used in both types of therapy.

Methods for administering the gene therapeutic composition of this invention to the body are not particularly limited. They may preferably be carried out by, for example, parental administration such as administration through injection. A variety of pharmaceutically acceptable excipients can be added to the composition.

The dosages for the gene therapeutic composition of this invention differ depending on the intended methods, the intended objects, etc., and one skilled in the art can readily make appropriate selection and optimization. Specifically, where administration by injection is used, preferably administration is done in a daily dose of from about 0.1 µg/kg to about 1,000 mg/kg, and more preferably in a daily dose of from about 1 µg/kg to about 100 mg/kg.

The nucleic acid carrier of this invention does not form precipitates upon complex formation with the therapeutic gene. Therefore, when the gene therapeutic composition of this invention is directly administered to the blood vessels, there is no risk of thrombus formation, thus allowing for efficient and precise administration of the therapeutic gene.

This invention will be described more concretely by way of examples; however, the invention is not to be limited by these examples.

### (EXAMPLES)

The compound numbers to be described in the following examples correspond to those denoted for the respective compounds in the reaction scheme of Fig. 1.

### (Example 1) Synthesis of 4-N-carbobenzoxy-DL-2,4-diaminobutyric acid (3)

DL-2,4-diaminobutyric acid dihydrochloride (1) (30 g, Sigma-Aldrich Corporation) was dissolved in 200 ml of water, to which 12.6 g of sodium hydroxide dissolved in 250 ml of water was added. To this was then added 101 g of [p-(benzyloxycarbonyl)phenyl]dimethylsulfonium methylsulfate (Z-DSP)(2) (Sanshin Chemical Industry, Co. Ltd.) at stirring over a period of about 20 minutes with ice cooling. During this period the pH was maintained at 9.4-9.7 by addition of an 8M aueous sodium hydroxide solution, and with ice cooling stirring was continued for additional 2 hours to yield the product as a precipitate. After the resulting product was filtered and washed with water and ethanol, it was dried to afford 28 g of the titled compound (3) (in 71% yield).

### (Comparative Example 1) Synthesis of 4-N-carbobenzoxy-DL-2,4-diaminobutyric acid (3)

DL-2,4-diaminobutyric acid dihydrochloride (1) (30 g, Sigma-Aldrich Corporation) was dissolved in 200 ml of water, to which 12.6 g of sodium hydroxide dissolved in 250 ml of water was added. To this was then added 101 g of Z-DSP (2) (Sanshin Chemical Industry, Co. Ltd.) at stirring over a period of about 20 minutes with ice cooling. During this period the pH was maintained at 11.1-11.5 by addition of an 8M aqueous sodium hydroxide solution, and with ice cooling stirring was then continued for 2 additional hours to yield the product as a precipitate. After the resulting product was filtered and washed with water and ethanol, it was dried to afford 15 g of the titled compound (3) (in 38% yield).

### (Example 2)

### 1. Synthesis of 4-N-carbobenzoxy-DL-2,4-diaminobutyric acid N-carboxy-anhydride (4)

Compound (3) obtained in Example 1 (12 g) was dissolved in tetrahydrofuran (THF, 400 ml). To this was added 5.5 g of triphosgene (bis(tricholoromethyl)carbonate, Tokyo Kasei Co. Ltd.) dissolved in 100 ml of THF, and it was stirred at 40 °C for 60 minutes. After removal of solvent under reduced pressure, hexane was added to the resulting crude product, after which it was cooled. After the supernatant hexane layer was removed by decantation, solvent was completely removed under reduced pressure. Ethyl acetate was added to the obtained residue for dissolution, and hexane was then added to form oily insoluble materials, which were cooled. After removing the supernatant by decantation, solvent was further thoroughly removed under reduced pressure. Diethyl ether was added to the resulting crude product to dissolve it and insoluble materials were removed by filtration. When the resulting filtrate was concentrated under reduced pressure' to a liquid quantity of 200 ml, colorless crystals precipitated. To these was further added hexane and cooled. The precipitated crystals were filtered and dried under reduced pressure to afford 7.5 g of the titled compound (4) (in 57% yield).

### 2. Synthesis of poly(4-N-carbobenzoxy-DL-2,4-diaminobutyric acid) (5)

4-N-carbobenzoxy-DL-diaminobutyric acid NCA (4) (1 g, 3.6 mmol) was dissolved in 19 ml of acetonitrile. Butylamine (n-BuNH₂) (4.38 mg, 0.06 mmol) was added as an initiator to the solution, which was then allowed to stand at 30 °C for 307 hours. After the resulting polymer was filtered and washed with acetonitrile and diethyl ether, it was dried under reduced pressure to afford 0.77 g of the titled compound (5) (the degree of polymerization=91%).

### 3. Synthesis of poly(DL-2,4-diaminobutyric acid) acetate (6)

Poly-4-N-carbobenzoxy-DL-diaminobutyric acid (5) (0.5 g) was dissolved in 2 ml of trifluoroacetic acid, to which a 25% hydrogen bromide/acetate solution was added and mixed with shaking. After allowing the solution to stand, the formed precipitates were twice washed with diethyl ether (ether removed by decantation). The resulting precipitates were sufficiently brought to dryness under reduced pressure. Sodium acetate and water were added to the obtained solids to produce a mixed solution. The mixed solution was dialyzed with running water using a dialysis tube for removing materials with a molecular weight of 1,000 or less. Then, centrifugation was carried out at 30,000 rpm for 2 hours to remove insoluble materials. The resulting solution was lyophilized to afford 0.34 g of poly-DL-diaminobutyric acid acetate (6) (in 91% yield).

### (Example 3) Coupling reaction (from pDBA acetate)

Cyanomethyl-1-thio-β-D-galactopyranoside (7) 23.5 mg, Sigma-Aldrich Corporation) was added to a 4-ml solution of 0.01M sodium methoxide methanol, which was allowed to stand at 25 °C for 24 hours. Subsequently, solvent was removed from the solution under reduced pressure, and 40 mg of diaminobutyric acid acetate (6) dissolved in 8 ml of tetraborate buffer (50 mM, pH 9.5) was added to the product and allowed to stand for 3 hours. The reaction solution was dialyzed with running water using a dialysis membrane with a fractionating molecular weight of 1,000. Then, centrifugation was carried out at 30,000 rpm for 2 hours to remove insoluble materials. The resulting solution was lyophilized to afford 31.2 mg of side chain galactose-modified poly-DL-diaminobutyric acid (9). The glycosylation percentage as determined by the anthronic-sulfuric acid reaction was 11%.

### (Example 4) Coupling reaction (from pDBA bromide) and Synthesis of poly(DL-2,4-diaminobutyric acid) bromide (6)

Poly-4-N-carbobenzoxy-DL-diaminobutyric acid (5) (90.0 mg) obtained Example 2-2 was dissolved in 0.5 ml of trifluoroacetic acid, to which a 25% hydrogen bromide/acetate solution (0.5 ml) was added and mixed with shaking. After allowing the solution to stand, the formed precipitates were twice washed with diethyl ether (ether removed by decantation). The resulting precipitates were sufficiently brought to dryness under reduced pressure. The obtained solids were dissolved in water. The solution was dialyzed with running water using a dialysis tube for removing materials with a molecular weight of 1,000 or less. Then, centrifugation was carried out at 30,000 rpm for 2 hours to remove insoluble materials. The resulting solution was lyophilized to afford 73.1 mg of poly-DL-diaminobutyric acid bromide (7)(in 99% yield).

Cyanomethyl-1-thio-β-D-galactopyranoside (7) (16.6 mg, Sigma-Aldrich Corporation) was added to a 2-ml solution of 0.01M sodium methoxide methanol, which was allowed to stand at 25 °C for 24 hours. Subsequently, solvent was removed from the solution under reduced pressure, and 25.5 mg of diaminobutyric acid bromide (6) dissolved in 4 ml of tetraborate buffer (50 mM, pH 9.5) was added to the product and allowed to stand for 3 hours. The reaction solution was dialyzed with running water using a dialysis membrane with a fractionating molecular weight of 1,000. Then, centrifugation was carried out at 30,000 rpm for 2 hours to remove insoluble materials. The resulting solution was lyophilized to afford 14.9 mg of side chain galactose-modified poly-DL-diaminobutyric acid (9). The glycosylation percentage as determined by the anthronic-sulfuric acid reaction was 9.7%.

### (Example 5) Gene transfer to HepG2 cells

### 1. Preparation of plasmid/pDBA complexes

A solution of a plasmid that encodes the luciferase gene and a solution of pDBA or the side chain galactose-modified polydiaminobutyric acid derivative (Gal-pDBA) as prepared in Examples 2-4 were, respectively, prepared at twice the desired concentration. The plasmid was an about 5.25-kb Picker gene control vector into which the luciferase gene had previously been inserted (Toyo Ink Co. Ltd.) and it will be abbreviated "Plasmid A" hereafter. Thirty minutes prior to administration, the nucleic acid carriers were added dropwise to the plasmid solution while stirring to prepare plasmid/pDBA complex solutions. PBS was used as solvent. Specifically, 25.0 µ g/ml plasmid solutions were prepared, and plasmid/pDBA complex solutions having a plasmid concentration of 12.5 µ g/ml were used as the gene transfer samples.

### 2. Gene transfer into HeLa cells and assay using the plasmid/pDBA complex solutions

HepG2 cells were inoculated onto a 12-well multi-well plate (Coaster Inc.) at 1x10⁵ cells/well one day prior to testing. The plasmid/pDBA complex solution was added to the HepG2 cells in the presence of 10% fetal bovine serum (Sanko Junyaku Co. Ltd.), and incubation was carried out at 37 °C for 4 hours. Medium was exchanged with a fresh culture medium and incubation continued for 48 hours. After washing with PBS twice, a cell lysis reagent (Toyo Ink Co. Ltd.) was added and freeze-thaw was carried out once. The cell lysate was recovered and subjected to centrifugation (12,000 rpm x 10 minutes). The luciferase activity of the supernatant was determined with a luminometer (LumitLB9501; Berthold Inc.) using a luciferase assay system (Toyo Ink Co. Ltd.). The protein concentration of the centrifuged supernatant was also determined with a microplate reader (Rainbow Themo; Tecan Inc.) using a protein assay kit (Bio-Rad Inc.).

Complex solutions were prepared for use in testing, where the weight ratios of Plasmid A to pDBA (plasmid/pDBA) were from 1/3 to 1/7.

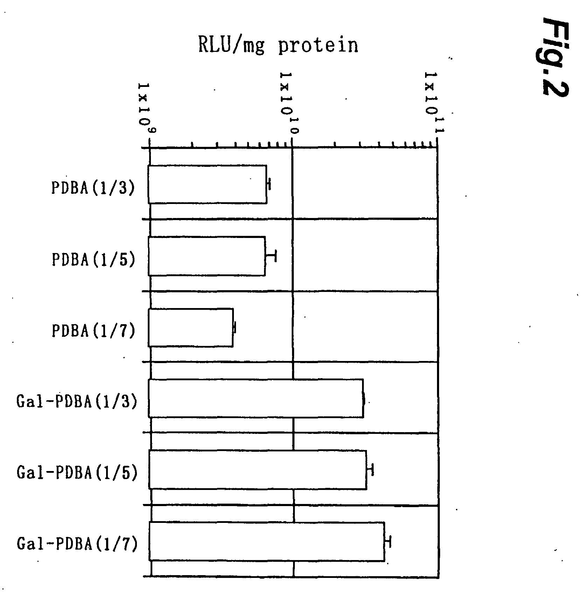

Fig. 2 shows the results of measurement of the activity of luciferase expressed. As is clear from these results of measurement, Gal-pDBA exhibited higher luciferase activity than did non-modified pDBA. Therefore, it was confirmed that when pDBA was glycosylated with a sugar according to this invention, the expression of luciferase markedly enhanced.

### (Example 6) Gene transfer to mice using pDBA complexes

### 1. Preparation of plasmid/pDBA complexes

A solution of Plasmid A and a solution of pDBA were prepared at twice the desired concentration. Thirty minutes prior to administration, the pDBA solutions were added dropwise to the plasmid solution while stirring to prepare plasmid/pDBA complexes solution. PBS was used as solvent. Specifically, 50.0 µ g/ml plasmid solutions were prepared, and plasmid/pDBA complex solutions having a plasmid concentration of 25 µ g/ml were used as the gene transfer samples.

Plasmid/pDBA complex solutions were prepared from Plasmid A and pDBA or Gal-pDBA as obtained in Examples 2-4 such that the weight ratios of plasmid/pDBA were from 1/3 to 1/9 (w/w). The complex solutions were administered to mice through their tail veins. The dosage of Plasmid A per mouse was 12.5 µg/0.5 ml. Two days after administration the gene transfer to the tissue was determined by measuring the luciferase activity found in the liver.

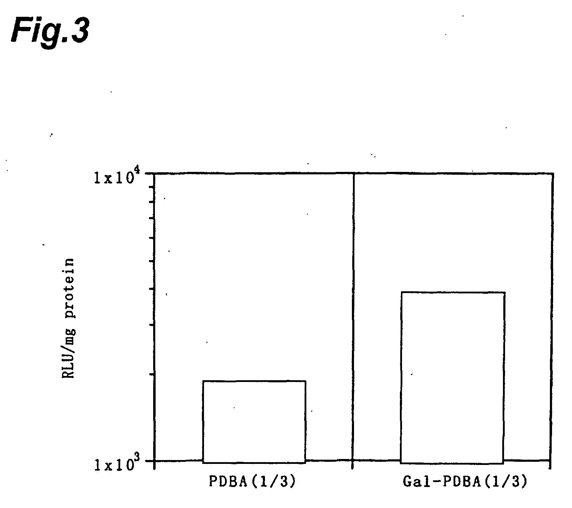

Fig. 3 shows the obtained results (data when the weight ratio of plasmid/pDBA was 1/3). Two days after administration in the group where Gal-pDBA was used as the nucleic acid carrier in the liver, about 2.5-fold luciferase activity was measured as compared to the group where pDBA was used as the nucleic acid carrier. This result demonstrates that when the nucleic acid carriers of the Examples are used for systemic administration, the gene can especially be transferred to the liver.

### Industrial Applicability

According to this invention, in the reaction for selectively protecting the 4-amino group of diaminobutyric acid with a carbobenzoxy group, the yield of the reaction and the purity of the product can be improved. Accordingly, the synthesis of highly pure carbobenzoxy-DBA will become possible in a short time and with a high yield.

The nucleic acid carriers of this invention can form complexes with a variety of therapeutic genes; and they enable the therapeutic genes to be transferred into cells efficiently and safely through different means as well as enable high expression of the genes in the cells.

It has been difficult to efficiently transfer a therapeutic gene into a particular organ or tissue by systemically administering the therapeutic gene with the use of a non-viral vector. However, the nucleic acid carriers of this invention can be used to enhance the gene transfer to the liver and allow for liver treatment in a concentrated manner.

## Claims

1. A peptide derivative represented by formula (1): 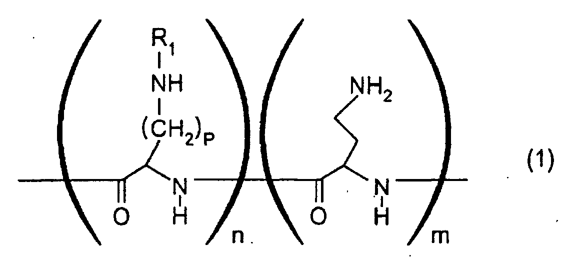 wherein R₁ is derived from one member selected from the group consisting of a monosaccharide, an oligosaccharide, a polysaccharide, a peptide, an oligopeptide, a polypeptide, an antibody and a cell-specific ligand, or is selected from the group consisting of an optionally substituted C₁-C₂₀ alkyl, an optionally substituted C₁-C₂₀ alkenyl, an optionally substituted C₇-C₄₅ aryl, an optionally substituted C₇-C₄₅ alkylaryl, an optionally substituted C₇-C₄₅ alkenylaryl, and an optionally substituted amino group wherein the substituent may be a moiety of the one member selected from the group consisting of a monosaccharide, an oligosaccharide, a polysaccharide, a peptide, an oligopeptide, a polypeptide, an antibody and a cell-specific ligand; "p" represents an integer of 1-5; "m" represents an integer of 2 or more; and "n" represents an integer of 2 or more, provided that "m + n" represents an integer of 300 or less, or a pharmaceutically acceptable salt thereof.

2. The polypeptide derivative or a pharmaceutically acceptable thereof according to claim 1, wherein R₁ is a radical represented by the formula of R₂-L-, wherein "L" is a linker selected from carbonyl, thiocarbonyl, imine or methylene which may further contain one or more methylene groups; and R₂ is a residual moiety of the R₁ radical as previously defined.

3. The polypeptide derivative or a pharmaceutically acceptable salt thereof according to claim 2, wherein L is a linker containing imine which may further contain one or more methylene groups; thus R₁ is a radical represented by formula (2) wherein "X" is 0 or a natural number of 20 or less and which is represented by formula (3).

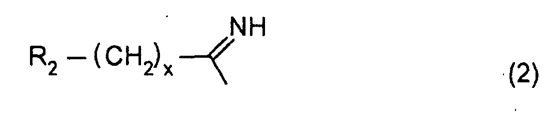

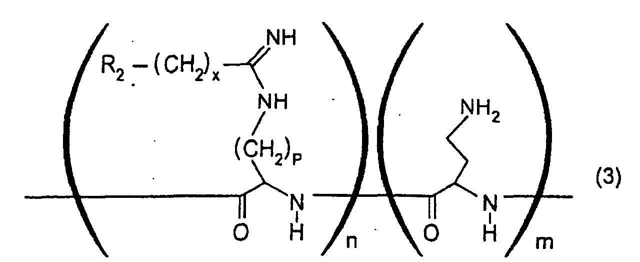

4. The polypeptide derivative or a pharmaceutically acceptable salt thereof according to claim 3, wherein "p" is 2 and which is represented by formula (4).

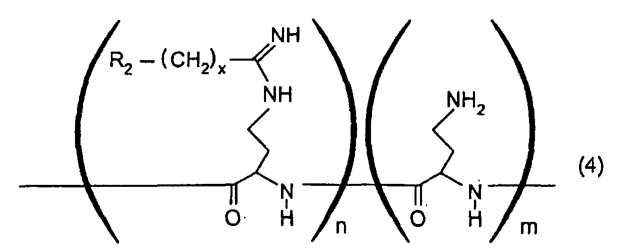

5. The polypeptide derivative or a pharmaceutically acceptable salt thereof according to claim 3 or 4, wherein R₂ is a radical derived from one member selected from the group consisting of a monosaccharide, an oligosaccharide, a polysaccharide, a peptide, an oligopeptide, a polypeptide, an antibody and a cell-specific ligand.

6. The polypeptide derivative or a pharmaceutically acceptable salt thereof according to claim 5, wherein R₂ is a thioglycosyl group, an O-glycosyl group, or a N-glycosyl group each of which is derived from one member selected from the group consisting of a monosaccharide, an oligosaccharide, and a polysaccharide.

7. The polypeptide derivative or a pharmaceutically acceptable salt thereof according to claim 6, which is represented by formula (5):

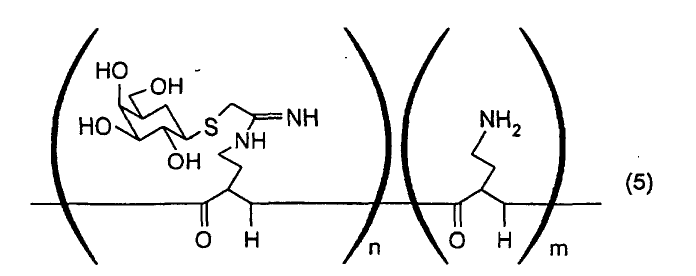

8. A nucleic acid carrier comprising the polypeptide derivative or a pharmaceutically acceptable salt thereof according to claim 1.

9. A gene therapeutic composition comprising the nucleic acid carrier according to claim 8 and a therapeutic gene.

10. A method of selectively protecting the 4-amino group of diaminobutyric acid with a carbobenzoxy group, comprising reacting diaminobutyric acid or a acid addition salt thereof with [p-(benzyloxycarbonyl)phenyl]dimethylsulfonium methylsulfate in a reaction inert medium at a pH of from 8 to 11.
